# EUROPÄISCHE PATENTSCHRIFT

(11) **EP 0 290 652 B1**
(45) Veröffentlichungstag und Bekanntmachung des Hinweises auf die Patenterteilung: **03.03.1993**
(21) Anmeldenummer: 87106977.9
(22) Anmeldetag: 14.05.1987
(51) Int. Cl.: A61B 5/103

(54) **Vorrichtung zur Messung der Beugekräfte des menschlichen Daumens**
Apparatus for the flexor-strength measurement of the human thumb
Dispositif pour mesurer la force de flexion des pouces humains

(43) Veröffentlichungstag der Anmeldung: 17.11.1988
(73) Patentinhaber: Weber, Hansjörg, Dr. med. habil., D-01662 Meissen (DE)
(72) Erfinder: Weber, Hansjörg, Dr. sc.med., DD-8250 Meissen (DD)
(74) Vertreter: Hoffmann, Klaus, Dr. rer. nat.

(56) Entgegenhaltungen:
- EP-A- 0 092 541
- DD-A- 204 206
- DE-A- 3 030 897
- US-A- 4 416 269

## Beschreibung

Die Erfindung betrifft eine Vorrichtung zur Messung der Beugekräfte des menschlichen Daumens bei allen Beugefunktionen und in Wirkungsrichtung der Kraft und für Hände unterschiedlicher Größe und bei biomechanisch weitgehend gleichen Bedingungen unter Anwendung einer Vorrichtung zum Messen von Beugekräften der menschlichen Hand oder einzelner Finger bekannter Konstruktion. Zusätzlich zu der bisher allein möglichen Messung der Beugekräfte des II. bis V. Fingers gestattet die Vorrichtung auch die Bestimmung der Beugekräfte des Daumens bei verschiedenen Beugefunktion jeweils in Wirkungsrichtung der Kraft. Die Vorrichtung gemäß der Erfindung ist für wissenschaftliche und praktische Fragestellungen anwendbar in verschiedenen Gebieten der Medizin, besonders aber in der Arbeitsmedizin, Sportmedizin, Chirurgie, Orthopädie, Neurologie, Physiotherapie und Rehabilitation.

Bekannt ist eine Vorrichtung, mit der Beugekräfte der Hand oder des II. bis V. Fingers bei verschiedenen Beugewinkeln der Finger ein- und derselben Hand oder bei biomechanisch gleichen Bedingungen für Hände unterschiedlicher Größe gemessen werden können (DD-PS 204 206).

Die Messung der Beugekräfte der Hand oder des II. bis V. Fingers einzeln wird dadurch ermöglicht, daß die Griffteile auf senkrecht zueinander stehenden Linien angeordnet und ihre Positionen auf diesen Linien frei wählbar sind und ein vom II. bis V. Fingers erfaßtes, vertikal auf die Höhe jedes Fingers einstellbares Griffteil im Wirkungseingriff mit einem Meßwandler steht.

Eine fehlerfreie Messung der Beugekraft des Daumens ist jedoch mit der bekannten Vorrichtung zum Messen von Beugekräften der menschlichen Hand oder einzelner Finger (DD-PS 204 206) nicht möglich.

Die bei der Entwicklung der Beugekraft der Hand (Handschluß) an der bekannten Vorrichtung erzeugte Kraft entspricht nicht der Beugekraft des Daumens, da die dem II. bis V. Finger gleich große Gegenkraft an dem als Widerlager dienenden horizontal beweglichen Griffteil nur zu einem Teil aktiv durch die Beugekraft des Daumens und des I. Mittelhandknochens und zum anderen Teil passiv und formschlüssig im Interdigitalbereich durch den I. und II. Mittelhandknochen, den Daumenballen und den Kleinfingerballen erzeugt wird. Außerdem entspricht dabei die Richtung der Beugekraft des Daumens nicht der Richtung der resultierenden Kraft des II. und V. Fingers, so daß auch aus diesem Grunde die Beugekraft des Daumens nicht die identische Gegenkraft zur resultierenden Kraft des II. und V. Fingers sein kann. Die Hauptkomponente der Beugekraft des Daumens wirkt in spitzem Winkel zur Längsachse des horizontal beweglichen Griffteiles ein.

Weiterhin ist ein Verfahren und eine Vorrichtung zur Ermittlung der isometrischen Spannung am Adductor-Pollicis-Muskel des Daumens bekannt (DE-OS 3 030 897).

Verfahren und Vorrichtung sind dabei so eingerichtet, daß die isometrische Kraft des Musculus adductor pollicis bei gestreckt fixiertem II. bis V. Finger und bei weitestmöglich abgespreiztem und festgelegten Daumen, d. h. bei maximaler Abduktion, gemessen wird. Das gegebene Verfahren und die Vorrichtung ermöglichen somit keine Bestimmung der Beugekräfte des Daumens bei verschiedenen Abduktions-, Flexions-, Volarabduktions- und Oppositionswinkeln des Daumens, was vor allem auch zur Messung der Daumenkraft bei in unterschiedlichen Positionen versteiften oder in unterschiedlichen Bereichen in der Bewegung eingeschränktem Daumen von Bedeutung ist. Es ist nicht mit Sicherheit anzunehmen, daß bei gestrecktem II. bis V. Fingers und weitestgehend gestrecktem und abgespreizten Daumen durch willkürliche Kontraktion die maximale Kraft des Musculus adductor pollicis entfaltet werden kann. Außerdem wird die Adduktion des Daumens in der in dem genannten Verfahren beschrieben Position bei willkürlicher Kontraktion nicht allein durch den Musculus adductor pollicis bewirkt.

Aufgabe der Erfindung ist es, eine Vorrichtung zum Messen der maximalen Flexions-, Adduktions-, Volarabduktions-und Oppositionskräfte des Daumens zu schaffen, die eine exakte Messung der Kräfte bei den genannten Beugefunktionen im umfassenden Sinne jeweils genau in Wirkungsrichtung der Kraft bei biomechanisch gleichen Bedingungen für Hände unterschiedlicher Größe und bei unterschiedlichen Winkelstellungen des Daumens in dan genannten Funktionen gegenüber der Hand erlaubt.

Erfindungsgemäß wird die Aufgabe gelöst durch eine Vorrichtung gemäß Anspruch 1.

Im folgenden wird ein Ausführungsbeispiel der Vorrichtung gemäß der Erfindung näher beschreiben:
Die beigefügte Zeichnung zeigt in:
- Fig. 1:: die schematische Vorderansicht der erfindungsgemäßen Vorrichtung,
- Fig. 2:: die schematische Seitenansicht der erfindungsgemäßen Vorrichtung entsprechend der Schnittlinie A - A.

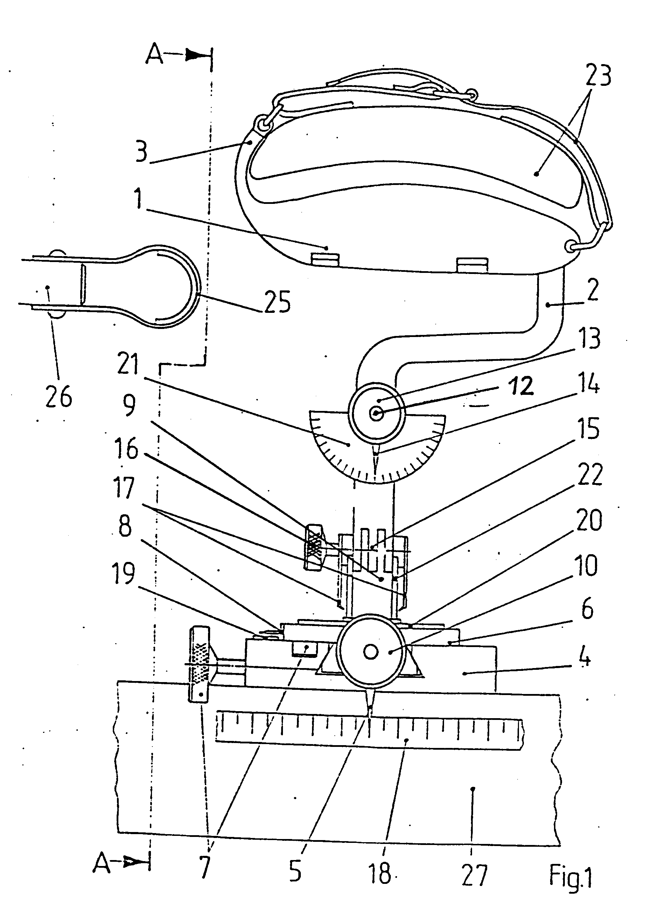

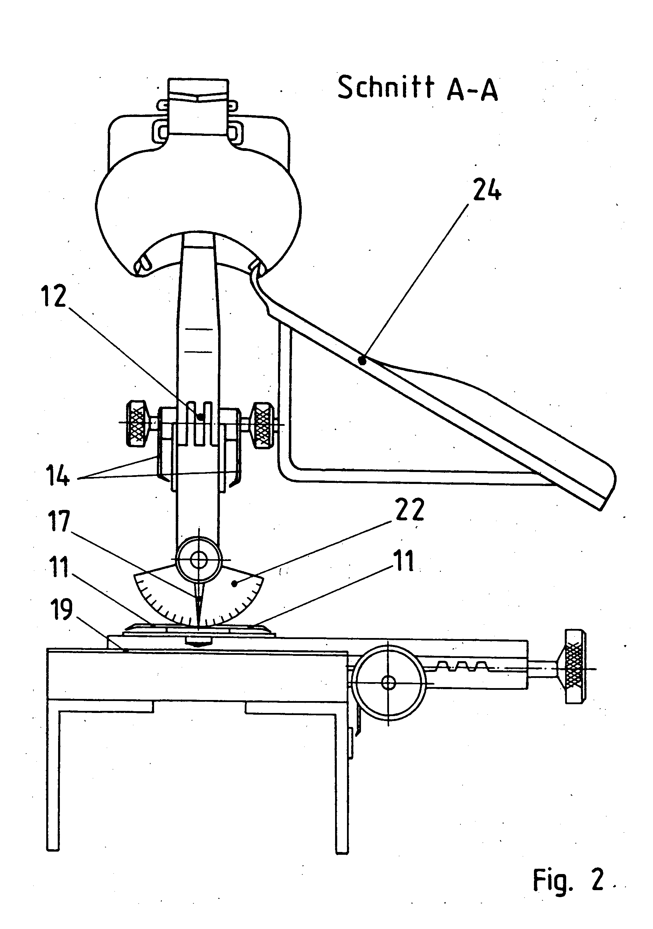

Ein Handgriff 1 ist über einen Bügel 2, Drehgelenk I 12, Drehgelenk II 15, Drehständer 9 und Quersupport 6 mit einem Support 4 verbunden. Die gegen ein zeichnerisch nicht dargestelltes Griffteil (DD-PS 204 206) ausgetauschte Vorrichtung zur Messung der Beugekräfte des menschlichen Daumens geht über den Support 4 mit einem zeichnerisch ebenfalls nicht dargestellten Widerlager der Vorrichtung 27 (DD-PS 204 206) eine lösbare Verbindung ein. Über das zeichnerisch nicht dargestellte Widerlager der Vorrichtung 27 wird der Support 4 in der Horizontalebene in Längsrichtung der Vorrichtung 27 verstellt. Dadurch kann der Längsabstand des Handgriffs 1 zum Fingerzuggriff 25 eingestellt werden. Der Längsabstand der Mittellinie des Drehständers 9 von dem dem Drehständer am weitesten angenäherten Punkt des Fingerzuggriffes 25 wird durch den Zeiger 5 am Support 4 auf dem horizontalen Maßstab 18 für den Support 4 angegeben. Der Querabstand der Mittellinie des Drehständers 9 von der Mittellinie des Fingerzuggriffes 25 und damit des Zugbolzens 26 ist von der Stellung des Quersupports 6 abhängig, die vom Zeiger 8 am Quersupport 6 auf dem horizontalen Maßstab 19 für den Quersupport 6 angezeigt wird. Die Einstellung der Position des Quersupports 6 erfolgt mit Hilfe des Vorschubs 7 des Quersupports 6. Die Winkelstellung des Handgriffs 1 gegenüber der Längsachse des Zugbolzens 26 in der Horizontalebene kann mit Hilfe des Drehständers 9 geändert werden. Zu diesem Zweck ist die Arretierung des Drehständers 10 zu lösen. Der Zeiger 11 am Drehständer 9 gibt die Winkelstellung des Handgriffs 1 zur Längsachse des Zugbolzens 26 auf einer horizontalen Skalenscheibe 20 an. Bei der in Figur 1 und 2 wiedergegebenen Stellung des Handgriffs 1 steht der Zeiger 11 am Drehständer 9 auf Ziffer "0" der horizontalen Skalenscheibe 20. Unter Bezug auf die durch diese Zeigerstellung gegebene Richtung ist der Handgriff 1 am Drehständer 9 sowohl nach rechts als auch nach links um 180° drehbar.

Die Winkelstellung des Handgriffs 1 zur Längsachse des Zugbolzens 26 in einer Vertikalebene I wird am Drehgelenk I 12 verändert. Dazu muß die Arretierung des Drehgelenks I 13 gelöst werden. Der Doppelzeiger I 14 zeigt auf dem vertikalen Skalensegment I 21 beiderseits des Handgriffs 1 den vertikalen Winkel des Handgriffs 1 zur Längsachse des Zugbolzens 26 an. Bei der in Figur 1 erkennbaren Stellung des Handgriffs 1 weist der Doppelzeiger I 14 auf Ziffer "0" des vertikalen Skalensegments I 21. In der Vertikalebene des Drehgelenks I 12 kann der Handgriff 1 im Uhrzeigersinn um + 90°, entgegen dem Uhrzeigersinn um - 90° verstellt werden.

Die Winkelstellung des Handgriffs 1 gegenüber der Horizontalebene kann außerdem mit Hilfe des Drehgelenkes II 15 in einer Vertikalebene II verändert werden, die sich im rechten Winkel zu der mit der Funktionsebene des Drehgelenkes I 12 identischen Vertikalebene I befindet. Zuvor ist die Arretierung 16 des Drehgelenkes II 15 zu lösen.

Der Doppelzeiger II 17 gibt auf dem vertikalen Skalensegment II 22 beidseits des Drehständers 9 die Winkelstellung des Handgriffs 1 gegenüber der Horizontalebene in der Vertikalebene II an. Die in Figur 2 dargestellte Position des Handgriffs 1 entspricht der Stellung "0" des Doppelzeigers II 17 auf dem vertikalen Skalensegment II 22.

Mit Hilfe des Supports 4 und Quersupports 6 zur Variation der Position des Handgriffs 1 gegenüber dem Fingerzuggriff 25 in der Horizontalebene, durch die Variation der vertikalen Position des Fingerzuggriffs 25 (DD-PS 204 206) sowie mittels des Drehständers 9, des Drehgelenks I 12 und des Drehgelenks II 15 zur Variation der Winkelstellungen des Handgriffs 1 ist es möglich, die maximale Kraftentfaltung an verschiedenen Meßpunkten des Daumens einschließlich des MP-Gelenkes in unterschiedlichen Winkelstellungen bei den verschiedenen Beugefunktionen des Daumens für unterschiedlich große Hände unter gleichen biomechanischen Bedingungen möglichst genau in Richtung der Achse des Zugbolzens 26 und damit ohne erheblich davon abweichende Kraftkomponenten quasi fehlerfrei zu messen.

Die horizontalen Maßstäbe 18 und 19 für Support 4 und Quersupport 6, die horizontale Skalenscheibe 20, das vertikale Skalensegment I 21 und das vertikale Skalensegment II 22 erlauben in Verbindung mit dem Zeiger 11 sowie dem Doppelzeiger I 14 und dem Doppelzeiger II 17, die Positionen und Winkelstellungen des Handgriffs 1 und der Unterarmstütze 24 in Millimetern und Winkelgrad anzugeben und damit auch die Stellung von Daumen, Hand und Unterarm bei Wiederholungsmessungen exakt zu reproduzieren.

Folgende Funktion der Vorrichtung werden zur Messung der Kräfte bei den verschiedenen Beugefunktionen des Daumens, von den in Figur 1 und 2 angegebenen Nullstellungen der Funktionen der Vorrichtung ausgehend, genutzt:
Zur Messung der Adduktionskräfte, die in der Handflächenebene bei verschiedenen Graden der Abduktion und an verschiedenen Meßpunkten des gestreckten Daumens erfaßt werden können, werden entsprechende Einstellungen des Supports 4, des Quersupports 6, des Drehständers 9, des Drehgelenkes I 12 und des Drehgelenkes II 15 vorgenommen, wobei der Winkel am Drehgelenk I 12 und Drehgelenk II 15 nach anfänglicher Einstellung unabhängig vom Abduktionswinkel konstant bleibt. Der Fingerzuggriff 25 ist nur einmalig zu Beginn der Messung auf die Höhe des in der Horizontalebene befindlichen Daumens einzustellen. Als Widerlager zur Erzeugung der Kraft dient hierbei das Widerlager 3 am Handgriff 1.

Zur Messung der Flexionskräfte im engeren Sinne, die an verschiedenen Meßpunkten des Daumens bei verschiedenen Graden der Flexion im IP-, MP- oder CM-Gelenk oder mehreren Gelenken zugleich in der Ebene unmittelbar unterhalb der Handfläche oder in einer Ebene erfolgen kann, die der Daumen bei Flexion zur Kuppe des Zeigefingers beschreibt, müssen die notwendigen Positionen und Winkel am Support 4, Quersupport 6, Drehständer 9, Drehgelenk I 12 und Drehgelenk II 15 eingestellt werden. Bei Messungen in einer Ebene bleiben die Winkel an den Drehgelenken I 12 und II 15 nach anfänglicher Einstellung unabhängig von den Flexionswinkeln konstant. Auch der Fingerzuggriff 25 wird nur einmal zu Beginn der Messung auf die Höhe des in der Horizontalebene befindlichen Daumens gebracht. Als Widerlager zur Erzeugung der Kraft ist bei der Messung der Flexionskräfte ebenfalls das Widerlager 3 am Handgriff 1 wirksam. Die Messung der Flexionskräfte am Grundglied und dem MP-Gelenk des Daumens bei maximaler Flexion in allen Gelenken wird dadurch möglich, daß der Handgriff 1 im Bereich des II. bis V. Fingers eine der Hohlhand angepaßte, dünnwandige Halbschale ist, und der U-förmige, mit dem Handgriff 1 lösbar verbundene Bügel 2 unter dem Handgriff 1 ebenfalls genügend Freiraum für die Unterbringung des Daumens läßt.

Zur Messung der Kräfte bei unterschiedlichen Graden der Volarabduktion und an verschiedenen Meßpunkten des Daumens wird die entsprechende Einstellung von Support 4, Quersupport 6, Drehgelenk I 12, Drehgelenk II 15, Drehständer 9 und Fingerzuggriff 25 vorgenommen. Dabei bleiben Drehgelenk II 15 und Drehständer 9 unabhängig vom Volarabduktionswinkel konstant eingestellt. Die Volarabduktion erfolgt in einer senkrecht zur Handfläche befindlichen Ebene, die der maximal abduzierte, gestreckte Daumen bei der Bewegung in Richtung Handfläche beschreibt. Zur Erzeugung der Daumenkräfte bei der Volarabduktion dient in den der maximalen Abduktion angenäherten Stellungen ein Riemen 23 mit Polster, in den der maximalen Volarabduktion angenäherten Stellungen das Widerlager 3 am Handgriff 1 zur Fixation der Hand.

Zur Messung der Daumenkräfte bei verschiedenen Graden der Opposition und an verschiedenen Meßpunkten des Daumens ist eine entsprechende Einstellung von Support 4, Quersupport 6, Drehständer 9, Drehgelenk I 12 und Drehgelenk II 15 erforderlich. Die vertikale Position des Fingerzuggriffs 25 und die Einstellungen von Drehgelenk I 12 und Dregelenk II 15 bleiben unabhängig vom Grad der Opposition konstant.

Die Oppositionsbewegung setzt sich aus der Volarabduktionsund der Flexions bewegung zusammen und ermöglicht der Daumenkuppe, die äußeren ulnar gelegenen Handflächengebiete zu erreichen. Zur Entwicklung der Daumenkraft bei Opposition bewirken der Handgriff 1 und teilweise das Widerlager 3 am Handgriff 1 die Fixation der Hand. Durch die beschriebene Konstruktion des Handgriffs 1 und des Bügels 2 wird die Messung der Daumenkraft auch bei Näherung an die maximale Opposition möglich.

## Patentansprüche

1. Vorrichtung zum Messen von Beugekräften des menschlichen Daumens bei allen Beugefunktionen und in Wirkungsrichtung der Kraft und für Hände unterschiedlicher Größe und bei biomechanisch weitgehend gleichen Bedingungen, bestehend aus:
- einem Support (4) und einem in einem Winkel von 90° dazu verschiebbar und einstellbar angeordneten Quersupport (6),
- einem Drehständer (9) mit daran befestigtem Handgriff (1), wobei der Handgriff (1) in zwei zueinander senkrechten Schwenkebenen mittels Drehgelenk I (12) und Drehgelenk II (15) schwenkbar und einstellbar und mittels des Drehständers (9) drehbar und einstellbar in der Bewegungsebene des Quersupports (6) gelagert ist und die Schwenkebene des Drehgelenkes II (15) sowie die Drehachse des Drehständers (9) senkrecht zur Bewegungsebene des Quersupports (6) angeordnet sind, und
- einem mit einem Meßwandler im Wirkungseingriff stehenden Fingerzuggriff (25) zur Aufnahme des Daumens,
so daß der horizontale und vertikale Winkel der Längsachse des Handgriffes (1) zur Längsachse des mit dem Meßwandler im Wirkungseingriff stehenden Fingerzuggriffs (25) durch den Drehständer (9) und das Drehgelenk I (12) und die Neigung des Handgriffs (1) gegenüber der Horizontalebene durch das Drehgelenk II (15) einstellbar sind und der Längs- und Querabstand des Handgriffs (1) bezüglich des Fingerzuggriffs (25) mit Hilfe des Längs- und Quersupports (4, 6) frei einstellbar ist.

2. Vorrichtung nach Anspruch 1, dadurch gekennzeichnet, daß der Handgriff (1) eine Halbschale geringer Wanddicke ist und ein Freiraum unter dem Handgriff (1) durch einen U-förmigen, mit dem Handgriff (1) lösbar verbundenen, Bügel (2) gebildet ist.

## Claims

1. A device for measuring bending forces of the human thumb in respect of all bending functions and in the direction of action of the force and for hands of different sizes and under biomechanically substantially identical conditions, comprising:
- a support (4) and a cross-support (6) arranged so as to be displaceable and adjustable at an angle of 90° in relation thereto,
- a swivel base (9) with a handle (1) attached thereto, where the handle (1) is mounted in such manner that in two swivel planes extending at right angles to one another it can be swivelled and adjusted by means of a swivel joint I (12) and a swivel joint II (15), and is rotatable and adjustable by means of the swivel base (9) in the plane of movement of the cross-support (6), and where the swivel plane of the swivel joint II (15) and the swivel axis of the swivel base (9) extend at right angles to the plane of movement of the cross-support (6) and
- a finger grip (25) which is in active engagement with a transducer and serves to accommodate the thumb,
so that the horizontal and vertical angle of the longitudinal axis of the handle (1) with respect to the longitudinal axis of the finger grip (25), which finger grip is in active engagement with the transducer, is adjustable by means of the swivel base (9) and the swivel joint I (12), and the inclination of the handle (I) with respect to the horizontal plane is adjustable by means of the swivel joint II (15), and the longitudinal and transverse spacing of the handle (1) with respect to the finger grip (25) is freely adjustable by means of the longitudinal- and cross-supports (4, 6).

2. A device as claimed in Claim 1, characterised in that the handle (1) is a half-shell with a small wall thickness, and a free space is formed below the handle (1) by a U-shaped member (2) detachably connected to the handle (1).

## Revendications

1. Dispositif pour mesurer la force de flexion des pouces humains, pour toutes les fonctions de flexion et dans la direction d'action de la force et pour des mains de tailles différentes et dans des conditions biomécaniques largement identiques, composé :
- d'un support (4) et d'un support transversal (6), déplaçable et réglable suivant un angle de 90 degrés par rapport à celui-ci,
- d'un bâti tournant (9), avec une poignée (1) fixée sur celui-ci, la poignée (1) étant supportée de façon à pouvoir pivoter et être réglée, dans deux plans de pivotement perpendiculaires entre eux, au moyen d'une articulation tournante I (12) et d'une articulation tournante II (15), et de façon à pouvoir tourner et être réglée, au moyen du bâti tournant (9), dans le plan de pivotement du support transversal (6), et le plan de pivotement de l'articulation tournante II (15) ainsi que l'axe de rotation du bâti tournant (5) étant disposés perpendiculairement au plan de déplacement du support transversal (6) et
- d'une prise de traction de doigt (25), placée en liaison fonctionnelle avec un convertisseur de mesure, pour supporter le pouce,
de sorte que les angles horizontal et vertical faits entre l'axe longitudinal de la poignée (1) et l'axe longitudinal de la prise de traction de doigt (25), reliée fonctionnellement au convertisseur de mesure, sont réglables au moyen du bâti tournant (9) et de l'articulation tournante I (12) et l'inclinaison de la poignée (1) par rapport au plan horizontal étant réglable au moyen de l'articulation tournante II (25) et les espacements longitudinal et transversal de la poignée (1) par rapport à la prise de traction de doigt (25) étant réglables librement à l'aide des supports longitudinal et transversal (4,6).

2. Dispositif selon la revendication 1, caractérisé en ce que la poignée (1) est une demi auge à faible épaisseur de paroi et un espace libre est formé sous la poignée (1), au moyen d'un étrier (2) en forme de U, relié amovible à la poignée (1).
